Europäisches Patentamt

European Patent Office

Office européen des brevets

Veröffentlichungsnummer: **0 295 397**

**A1**

# EUROPÄISCHE PATENTANMELDUNG

Anmeldenummer: **88106622.9**

Int. Cl.⁴ **A61L 27/00**

Anmeldetag: **26.04.88**

Priorität: **17.06.87 CH 2282/87**

Veröffentlichungstag der Anmeldung:
**21.12.88 Patentblatt 88/51**

Benannte Vertragsstaaten:
**AT BE DE ES FR GB IT NL SE**

Anmelder: **GEBRÜDER SULZER AKTIENGESELLSCHAFT**
**Zürcherstrasse 9**
**CH-8401 Winterthur(CH)**

Erfinder: **Nüesch, Peter**
**im Gaschlieser 807**
**CH-9496 Balzers(CH)**
Erfinder: **Semlitsch, Manfred, Dr.**
**Endlikerstrasse 82**
**CH-8400 Winterthur(CH)**

Vertreter: **Dipl.-Ing. H. Marsch Dipl.-Ing. K.**
**Sparing Dipl.-Phys.Dr. W.H. Röhl**
**Patentanwälte**
**Rethelstrasse 123**
**D-4000 Düsseldorf 1(DE)**

Metallisches Implantat.

Eine Hartstoff-Beschichtung, die mindestens auf Teile der Oberfläche von Implantaten für den menschlichen oder tierischen Körper als Schutzschicht aufgebracht ist, bestehend aus Titankarboxid [Ti(N,C,O)].

Gegenüber bisherigen Hartstoff-Beschichtungen für Implantate weist diese Schicht eine deutlich verbesserte Benetzbarkeit mit wasserhaltigen Flüssigkeiten auf, woraus gute tribologische Eigenschaften gegenüber Kunststoffen, beispielsweise bei Gelenkimplantaten, resultieren. Ausserdem ist die Beschichtung duktiler, was ihre Haftfestigkeit und Verletzbarkeit auf dem Substrat günstig beeinflusst.

EP 0 295 397 A1

## Metallisches Implantat

Die Erfindung betrifft ein für den menschlichen oder tierischen Körper bestimmtes metallisches Implantat, das aus einer Legierung auf Eisen-, Kobalt- oder Titanbasis oder aus Reintitan besteht und mindestens teilweise mit einer Hartstoffschicht auf Titanbasis beschichtet ist.

Es ist bekannt, mindestens Teile der Oberfläche von Implantaten mit Schutzschichten zu versehen, um ihre Verschleissfestigkeit und/oder Korrosionsbeständigkeit zu erhöhen. Für diese Schutzschichten sind vor allem Hartstoffe auf Titanbasis verwendet worden. Beispielsweise hat man als Paarung für aufeinander bewegte metallische Gleitflächen eines Gelenkimplantates die eine Fläche vorzugsweise mit Titankarbid (TiC) und die Gegenfläche mit Titannitrid (TiN) beschichtet (EP-A-159 410).

In der Praxis hat sich nun gezeigt, dass die bisher verwendeten Hartstoff-Beschichtungen als Gleitpartner gegenüber Kunststoffen - oder anderen, verglichen mit der Beschichtung, relativ weichen Stoffen, wie Knochengewebe oder einem mindestens im wesentlichen aus einem Polymer bestehenden Knochenzement - weniger gut geeignet sind, da sie mit wasserhaltigen Flüssigkeiten nicht gut benetzbar sind und ausserdem relativ spröde sind.

Aufgabe der Erfindung ist es daher, Metallimplantate mit Hartstoff-Beschichtungen zu versehen, die als Gleitpartner gegenüber Polymeren, insbesondere gegenüber Polyethylen (PE) der für Gelenkimplantate üblichen Spezifikation, besser geeignet sind als die bisher verwendeten Hartstoffe. Erfindungsgemäss wird diese Aufgabe dadurch gelöst, dass die Hartstoffbeschichtung aus Titan-Nitrokarboxid [Ti(N,C,O)] besteht.

Durch den Einbau von Sauerstoff werden die Titankarbonitride erheblich duktiler und haben eine deutlich verbesserte Benetzbarkeit mit wasserhaltigen Flüssigkeiten. Daraus resultieren gute tribologische Eigenschaften. Weiterhin sind mit der neuen Beschichtung versehene Oberflächen kratzfester als ungeschützte Metalloberflächen, die einen vergleichbaren Polymerabrieb bewirken. Dadurch wird indirekt die Gefahr von Beschädigungen der polymeren Gegenlauffläche durch verkratzte Metalloberflächen verringert.

Die neue Beschichtung hat sich darüber hinaus auch als Verschleissschutz des Metall-Substrates bei Verankerungskomponenten von Implantaten bewährt, die kleine Relativbewegungen (Mikrobewegungen) in einem Zementbett oder gegenüber dem sie umgebenden Knochengewebe ausführen. Ein Metallabrieb, der zu verstärkten Korrosionserscheinungen führen könnte, kann so verhindert werden.

Als vorteilhaft hat es sich erwiesen, wenn die Härte der Hartstoff-Beschichtung mindestens 600 und höchstens 1500 Vickerseinheiten ist; dies lässt sich beispielsweise erreichen, wenn in der Hartstoff-Beschichtung, in Atomprozent gemessen, die Summe der Stickstoff- und der Kohlenstoffatome geringer ist als der Anteil der Sauerstoffatome, wobei die Härte durch Variation der Stickstoff-, Kohlenstoff- und Sauerstoff-Anteile in gewissem Umfang variiert und gesteuert werden kann.

Insbesondere haben sich Zusammensetzungen bewährt, bei denen die Hartstoff-Beschichtung, gemessen in Atomprozent, enthält: Titan 40 - 45, insbesondere 42; Stickstoff 6 - 16, insbesondere 8; Kohlenstoff 4 - 12, insbesondere 6 und Sauerstoff 30 - 48, insbesondere 44.

Die Herstellung der Hartstoff-Schichten auf dem Metallimplantat kann nach einem geeigneten bekannten Herstellungsverfahren, z.B. nach dem CVD- oder dem PVD- (chemical bzw. physical vapor deposition)-Verfahren erfolgen. Die dabei aufgetragenen Schichten haben vorteilhafterweise Dicken von 0,5 bis 10 $\mu$m.

Im folgenden wird die Erfindung anhand von Ausführungsbeispielen näher erläutert.

### Beispiel 1:

Das Substrat ist ein Gelenkkugelkopf aus einer Eisen-, Kobalt- oder Titanbasis Legierung; dieser Kugelkopf soll mit Hilfe eines der Herstellungsverfahren mit einer 1 bis 5 $\mu$m dicken [Ti(N,C,O)]-Schicht versehen werden.

Zunächst wird die als Gleitfläche in einer Kunststoff-Pfanne "laufende" Kugel-Oberfläche auf bekannte Weise hochglanzpoliert (Rauheitsklasse N4 oder besser). Das polierte Substrat wird in eine PVD-Beschichtungsanlage mit Titankathode eingebracht und im Plasma einer Edelgas-Atmosphäre in Anwesenheit von Stickstoff ($N_2$), Sauerstoff ($O_2$) und Kohlenmonoxid (CO) nach dem bekannten Verfahren der Magnetron-Zerstäubungstechnik mit Ti(N,C,O) beschichtet. Um die nachstehend genannte Zusammensetzung der Hartstoff-Schicht zu erreichen, betragen die relativen Anteile der drei benetzten Gase in der "Atmosphäre" der Anlage, - in Volumenprozent - Stickstoff 20, Kohlenmonoxid 50 und Sauerstoff 30.

Die Beschichtung dauert etwa 3 Stunden., wobei sich eine Schichtdicke für die Hartstoff-Schicht von etwa 2 bis 3 $\mu$m aufbaut; die Zusam-

mensetzung der Hartstoff-Schicht beträgt, in Atomprozent. Titan 42, Stickstoff 8, Kohlenstoff 6 und Sauerstoff 44. Für die Härte der Schicht werden etwa 600 HV gemessen.

## Beispiel 2:

Der Verankerungsteil einer Femurkopfprothese ist mindestens in seinem im Knochen - oder einen Knochenzementbett -gelagerten Bereich mit einer Hartstoff-Schicht aus Ti(N,C,O) zu versehen, deren Härte bei etwa 1500 HV liegt.

Der in bekannter Weise mit einer Oberflächenstruktur versehene Schaft wird in die PVD-Beschichtungsanlage mit Titankathode des Beispiels 1 eingebracht und wiederum im Plasma einer Edelgas-Atmospäre in Anwesenheit von Stickstoff. Sauerstoff und - als Kohlenstofflieferant - Azetylen ($C_2H_2$) nach dem gleichen Verfahren wie die Gleitfläche des Gelenkkopfes nach dem ersten Beispiel mit Ti(N,C,O) beschichtet.

Um die gewünschte Härte zu gewährleisten, ist es notwendig, den relativen Sauerstoff-Anteil in der Schicht zugunsten der beiden anderen Partner Stickstoff und Kohlenstoff zu erniedrigen. Im vorliegenden Fall ist daher eine Zusammensetzung der Schicht, in Atomprozent, von Ti 42, N 16, C 12 und O 30 erforderlich. Das neben dem Edelgas-Plasma vorhandene Gasgemisch in der Anlage hat dafür eine relative Zusammensetzung, in Volumenprozent. 20 $N_2$. 50 $C_2H_2$ und 30 $O_2$.

Beschichtungsdauer und Schichtdicken entsprechen den im Beispiel 1 angegebenen Werten. Die Härte der Schicht auf dem Prothesenschaft beträgt, wie gefordert, etwa 1500 HV.

## Ansprüche

1. Für den menschlichen oder tierischen Körper bestimmtes metallisches Implantat, das aus einer Eisen-, Kobalt-oder Titanbasis Legierung oder aus Reintitan besteht und mindestens teilweise mit einer Hartstoff-Schicht auf Titanbasis beschichtet ist, dadurch **gekennzeichnet,** dass die Hartstoff-Beschichtung aus Titan-Nitrokarboxid [Ti(N,C,O)] besteht.

2. Implantat nach Anspruch 1. dadurch gekennzeichnet, dass in der Hartstoff-Beschichtung, in Atomprozent gemessen, die Summe der Stickstoff-(N)- und der Kohlenstoff(C)-Atome geringer ist als der Anteil der Sauerstoff(O)-Atome.

3. Implantat nach Anspruch 2. dadurch gekennzeichnet, dass die Zusammensetzung der Hartstoff-Beschichtung, gemessen in Atomprozent, beträgt:

Titan (Ti)    40 - 45;
Stickstoff (N)    6 - 16:
Kohlenstoff (C)    4 - 12;
Sauerstoff (O)    30 - 48.

4. Implantat nach Anspruch 3. dadurch gekennzeichnet, dass die Zusammensetzung der Hartstoff-Beschichtung. gemessen in Atomprozent, ist:

Titan (Ti)    42;
Stickstoff (N)    8;
Kohlenstoff (C)    6;
Sauerstoff (O)    44.

5. Implantat nach Anspruch 1. dadurch gekennzeichnet, dass die Dicke der Hartstoff-Beschichtung 0,5 - 10 $\mu$m beträgt.

6. Implantat nach Anspruch 1. dadurch gekennzeichnet, dass die Hartstoff-Beschichtung auf die Gleitfläche eines Gelenkimplantats aufgebracht ist, deren Gegenlauffläche aus Kunststoff, insbesondere aus Polyethylen (PE), besteht.

7. Implantat nach Anspruch 1. dadurch gekennzeichnet, dass die Härte der Hartstoff-Beschichtung mindestens 600 und höchstens 1500 Vickers (HV) ist.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | EP-A-0 222 717 (INSTITUTE FOR APPLIED BIOTECHNOLOGY) * Ansprüche 1,4 * | 1 | A 61 L 27/00 |
| Y | GB-A-2 145 117 (DNEPROPETROVSKY MADITSINSKY INSTITUT) * Ansprüche 1,2 * | 2-7 | |
| Y,D | EP-A-0 159 410 (SULZER) * Seite 1, Zeilen 16-18; Ansprüche 1,2,4 * | 2-7 | |
| A | DE-A-1 943 801 (INSTITUT R. STRAUMANN) * Anspruch 1 * | | |

| RECHERCHIERTE SACHGEBIETE (Int. Cl.4) |
|---|
| A 61 L 27/00 |

Der vorliegende Recherchenbericht wurde für alle Patentansprüche erstellt

| Recherchenort | Abschlußdatum der Recherche | Prüfer |
|---|---|---|
| DEN HAAG | 05-09-1988 | PELTRE CHR. |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus andern Gründen angeführtes Dokument
......................................................................
& : Mitglied der gleichen Patentfamilie, übereinstimmendes
    Dokument

EPO FORM 1503 03.82 (P0403)